(19) **Europäisches Patentamt**

**European Patent Office**

**Office européen des brevets**

(11) Veröffentlichungsnummer: **0 311 065 B1**

(12) **EUROPÄISCHE PATENTSCHRIFT**

(45) Veröffentlichungstag der Patentschrift: **02.02.94**

(21) Anmeldenummer: **88116522.9**

(22) Anmeldetag: **06.10.88**

(51) Int. Cl.⁵: **A61K 9/22**

(54) **Implantierbares, biologisch abbaubares Wirkstofffreigabesystem.**

(30) Priorität: **09.10.87 DE 3734223**

(43) Veröffentlichungstag der Anmeldung:
**12.04.89 Patentblatt 89/15**

(45) Bekanntmachung des Hinweises auf die
Patenterteilung:
**02.02.94 Patentblatt 94/05**

(84) Benannte Vertragsstaaten:
**AT BE CH DE ES FR GB GR IT LI LU NL SE**

(56) Entgegenhaltungen:
**EP-A- 0 094 513**
**EP-A- 0 251 476**
**FR-A- 2 126 270**
**US-A- 3 317 394**

(73) Patentinhaber: **BOEHRINGER INGELHEIM KG**

**D-55216 Ingelheim(DE)**
(84) Benannte Vertragsstaaten:
**BE CH DE ES FR GR IT LI LU NL SE AT**

(73) Patentinhaber: **BOEHRINGER INGELHEIM IN-
TERNATIONAL GmbH**

**D-55216 Ingelheim(DE)**
(84) Benannte Vertragsstaaten:

**GB**

(72) Erfinder: **Stricker, Herbert, Prof.Dr.**
**Richard Lenel-Weg 13**
**D-6903 Neckargemünd(DE)**
Erfinder: **Entenmann, Günther, Dr.**
**Schützenpfad 16**
**D-6507 Ingelheim am Rhein(DE)**
Erfinder: **Kern, Otto, Dr.**
**Rinderbachstr. 33**
**D-6507 Ingelheim am Rhein(DE)**
Erfinder: **Mikhail, Michel, Dr.**
**Am Landgraben 14**
**D-6507 Ingelheim am Rhein(DE)**
Erfinder: **Zierenberg, Bernd, Dr.**
**Goethestr. 1**
**D-6530 Bingen(DE)**

**Beschreibung**

Die Erfindung betrifft ein implantierbares, biologisch abbaubares Wirkstoffreigabesystem.

Aus dem stand der Technik sind zahlreiche Wirkstoffreigabesysteme bekannt, die implantier- oder injizierbar sind. Solche Systeme werden bevorzugt dann angewendet, wenn ein Wirkstoff über einen längeren Zeitraum verabreicht werden soll und eine perorale Applikation nicht möglich, bzw. nicht verläßlich, bzw. nicht sinnvoll ist. FR-A-2 126 270 beschreibt ein Retardpräparat, welches zusätzlich zum Wirkstoff noch Polylactid und Weichmacher (PE6 oder mono-, oligo-Milchsäure enthalten kann. Porenbildner wird nicht beigegeben. Neben einer Anwendung im Humanbereich sind parenterale Applikationsformen bei der Tieraufzucht bzw. Therapie von Tiererkrankungen von besonderem Interesse. Die herkömmliche Dosierung von Arzneimitteln über die Zumischung zum Futter hat den gravierenden Nachteil, daß die aufgenommene Arzneimittelmenge nicht befriedigend genau ist.

Implantierbare Wirkstoffreigabesysteme sollten die folgenden Kriterien erfüllen:
Der Wirkstoff sollte über einen längeren Zeitraum in gleichbleibender Geschwindigkeit abgegeben werden, das Implantat sollte in einem angemessenen Zeitraum abgebaut werden, so daß eine operative Entfernung des Implantats nach der Wirkstoffabgabe entfällt. Weiterhin ist es von Vorteil, wenn die Wirkstoffreigabe des Trägers variabel einstellbar ist, damit die Freisetzungsrate sowohl dem Wirkstoff als auch der Therapie angepaßt werden kann.

Es ist die Aufgabe der vorliegenden Erfindung ein implantierbares, biologisch abbaubares Wirkstoffreigabesystem zur Verfügung zu stellen, das den Wirkstoff über einen längeren Zeitraum in nahezu gleichbleibender Geschwindigkeit freigibt und in einem vertretbaren Zeitraum abgebaut ist.

Die Aufgabe wird durch ein Implantat aus einem Trägermaterial auf der Basis von Poly-D,L-Lactid mit definierten Anteilen an Zusätzen gelöst. Geeignete Zusätze sind pharmakologisch verträgliche Essigsäureester, mit einem Anteil bis zu 10 % und/oder einer biologisch abbaubaren Polymilchsäure, mit einem Anteil bis zu 60 % und/oder suspendierten, wasserlöslichen Porenbildnern, wie z.B. Lactose, mit einem Anteil bis zu 50 Gew. %.

Poly-D,L-Lactide sind in einem weiten Molekulargewichtsbereich bekannt. Für das erfindungsgemäße Implantat sind bevorzugt die mittelmolekularen Poly-D,L-Lactid-Typen geeignet, die eine inhärente Viskosität zwischen 0.15 und 4.5 (gemessen in Chloroform bei 25 °C, c = 100 mg/100 ml) aufweisen.

In einer bevorzugten Ausführungsform besteht das Trägermaterial des erfindungsgemäßen Implantats aus Poly-D,L-Lactid.

In einer anderen Ansführungsform besteht das erfinungsgemäße Implantat aus einem Copolymer aus D,L-Lactid und Glycolid, wobei jedoch der Anteil des Glycolids an dem Copolymerisat 50 Gew.% nicht überschreiten sollte.

Überraschenderweise wurde gefunden, daß die Abbaurate des Implantats durch einen definierten Gehalt an Essigsäureester, welcher auch nach längerer Lagerung quantitativ im Polymer verbleibt, gesteuert werden kann. Dies ist von entscheidender Bedeutung, da einerseits das Implantat ausreichend rasch abgebaut werden soll, andererseits ein zu schneller Abbau des Implantats zu einer unkontrollierten Freisetzung des Wirkstoffes führt. Der Gehalt an Essigsäureester kann bis zu 10 % betragen, wobei ein steigender Anteil Essigsäureester den Abbau des Poly-D,L-Lactids beschleunigt. Günstig ist eine Wirkstoffreisetzung entsprechend Halbwertszeiten zwischen 3 und 60 Tagen und ein ab da einsetzender Abbau des Implantats innerhalb von ca. 120 Tagen. Im Einzelfall können selbstverständlich auch kürzere Freisetzungs- und Abbauraten von Vorteil sein.

Überraschenderweise wurde gefunden, daß der Zusatz von Essigsäureester zwar die Abbaurate des Implantats beeinflußt, aber auf die Wirkstoffreigabe keinen nennenswerten Einfluß hat.

Essigsäureester im Sinne der Erfindung sind die Alkylester der Essigsäure, wie z.B. der Methyl-, Ethyl-, n-Propyl, iso-Propyl-, n-Butyl, iso-Butyl-, tert.-Butyl-, n-Pentyl-, sec.-Pentyl-, iso-Pentyl und der tert.-Pentylester. Besonders bevorzugt ist der Essigsäureethylester, auch als Essigester bezeichnet.

In einer weiteren Ausführungsform kann das erfindungsgemäße Implantat auch niedermolekulare Polymere, wie z.B. Poly(L-Milchsäure), Poly(D-Milchsäure), Poly(D,L-Milchsäure), Poly(glycolsäure), Poly(L-Milchsäure-co-Glycolsäure), Poly(D-Milchsäure-co-Glycolsäure), Poly(D,L-Milchsäure-co-Glycolsäure) enthalten. Bevorzugt werden Poly(L-Milchsäure) und Poly(D,L-Milchsäure). Die Molekulargewichte (bestimmt durch Endgruppentitration) betragen 500 bis 5000, vorzugsweise 1500 bis 2500.

Diese Zusätze ermöglichen für sich allein oder in Kombination mit einem Essigsäureester ebenfalls eine Steuerung der Abbaurate des Implantats.

Eine Beeinflussung der Wirkstoffreigabe kann auf verschiedene Weise vorgenommen werden:
a) Zusatz eines Porenbildners, wie z.B. Lactose u.a.,
b) Zustand des Wirkstoffs (gelöst, suspendiert, Teilchengröße),

c) Verarbeitungsform des Trägers (monolithisch, polydispers, Schichtaufbau).

Neben Verbindungen, die die Abbaurate des Trägermaterials beeinflussen, enthält das erfindungsgemäße Implantat auch Stoffe in Form von Porenbildnern, die eine Steuerung der Wirkstofffreigabe ermöglichen. Erfindungsgemäß geeignete Porenbildner sind beispielsweise wasserlösliche pharmazeutisch akzeptable Monosaccharide und Disaccharide. Bevorzugt wird Lactose, geeignet sind jedoch auch Glucose, Fructose, Xylose, Galactose, Sucrose, Maltose, Saccharose und verwandte Verbindungen wie Mannit, Xylit, Sorbit. Andere geeignete Hilfsstoffe sind Salze wie Lactate, Glyconate oder Succinate des Natriums, Kaliums oder Magnesiums.

Eine rasche Freisetzung der Wirksubstanz von Anfang an (nach erfolgter Implantation) aus dem Trägermaterial wird dann erreicht, wenn die Freisetzungsgeschwindigkeit des Porenbildners sehr viel größer ist als die der Wirksubstanz. Dies ist beispielsweise bei guter Löslichkeit und kleiner Partikelgröße des Porenbildners, z.B. Lactose, der Fall.

Eine verspätet beschleunigte Freisetzung der Wirksubstanz wird dann erreicht, wenn die Löslichkeit des Porenbildners sehr viel kleiner ist als die der Wirksubstanz; so z.B. bei schlechter Wasserlöslichkeit des Porenbildners. Durch die verspätet beschleunigte Freisetzung der Wirksubstanz wird erreicht, daß der lineare Freigabeverlauf der Wirksubstanz auch bei längerer Applikation gewährleistet bleibt.

Unter Ausnutzung der beschriebenen Parameter können Implantate hergestellt werden, die sowohl eine individuell einstellbare Freigabe- wie auch Abbaurate aufweisen.

Das erfindungsgemäße monolithische Implantat kann in Form von Stäbchen bzw. als schlauchförmiger Körper injiziert bzw. implantiert werden. Zweckmäßigerweise sind die Stäbchen so bemessen, daß sie noch mit einer Injektionsnadel oder einem Trokar implantiert werden können. Beispielsweise beträgt die Länge eines Stäbchens ca. 3 cm, und der Durchmesser ca. 2,8 mm.

Bevorzugt sind die nachfolgend beschriebenen Ausführungsformen:

Typ A: massive Stäbchen
Typ B: gerollte Filme
Typ C: Ummantelte Stäbchen
Typ D: Schlauchförmige Körper
Typ E: Ummantelte schlauchförmige Körper

Alle Ausführungsformen des erfindungsgemäßen Implantats können mehrschichtig aufgebaut und beispielsweise nach folgendem Verfahren hergestellt werden.

In dem gelösten Polymer, z.B. mit Essigester als Lösungsmittel, wird der Wirkstoff suspendiert und die erfindungsgemäßen Zusätze beigemengt. Wenn es gewünscht wird, können dem gelösten Polymer neben dem Wirkstoff und den Zusätzen noch weitere pharmazeutische Hilfsstoffe zugesetzt werden. Danach wird die Suspension auf einer Fläche ausgegossen und zu einem Film getrocknet. Hierbei werden die Trocknungsbedingungen so eingestellt, daß die gewünschte Restmenge an Lösungsmittel in dem Polymer verbleibt im allgemeinen zwischen 1 und 7 %. Die getrockneten Filme haben eine Schichtdicke zwischen 30 und 1000 $\mu$, bevorzugt ca. 100 $\mu$. Geräte und Verfahren zur Herstellung solcher Filme sind dem Fachmann bekannt und bedürfen keiner weiteren Ausführungen. Es ist selbstverständlich, daß der Trocknungsprozeß mit einer gewissen Sorgfalt (langsam, geringe Temperatur- und Vakuum-Feuchtigkeitsschwankungen) durchgeführt werden muß, damit die Filme plan bleiben.

Mehrschichtige Filme können durch ein erneutes Aufbringen von Polymerlösung (mit oder ohne Wirkstoff) erhalten werden.

Nachdem der Film getrocknet ist, wird er zu Stäbchen der gewünschten Länge geschnitten.

Stäbchen des Typs B bestehen aus einem oder mehreren aufgerollten ein- oder mehrschichtigen Polymerfilmen.

Das erfindungsgemäße Implanat des Typs B wird ebenfalls aus wirkstoffhaltigen Filmen hergestellt, wobei jedoch die Dicke der Filme wesentlich geringer ist, im allgemeinen zwischen 30 und 500 $\mu m$, bevorzugt 70 bis 90 $\mu m$. Nach dem Trocknen werden die Filme geschnitten und zu Stäbchen gewünschten Durchmessers, bis ca. 3 mm, gerollt, die dann auf die gewünschte Länge geschnitten werden. Die Stäbchen können so aufgerollt werden, daß der Kern einen Hohlraum enthält. Bei dem Laminat des Typs B können auch mehrere Filme übereinandergelegt oder bevorzugt übereinander gegossen werden, die dann zu einem Stäbchen gerollt werden. Durch die Kombination mehrerer Filmschichten können in einfacher Weise Wirkstoffe kombiniert werden und Schichten unterschiedlicher Wirkstoffkonzentration hergestellt werden. Die einzelnen Schichten können verschiedene Freisetzungsgeschwindigkeiten aufweisen.

Neben einer alternierenden Schichtfolge ist es auch möglich, zuerst einen aufgerollten Kern zu bilden und anschließend außen weitere Filmschichten aufzubringen.

Durch die Verwendung von Filmschichten mit unterschiedlicher Freisetzungscharakteristik können mit einem Implantat auch verschiedene Wirkstoffe in zeitlich voher bestimmter Reihenfolge abgegeben werden.

Es ist nicht unbedingt erforderlich, daß alle Filmschichten Wirkstoffe enthalten.

Bei der Herstellung des erfindungsgemäßen Implantats des Typs B sollten die Filme einen relativ hohen Gehalt an Restlösungsmittel aufweisen (ca. 10.%), wenn sie gerollt werden. Hierdurch wird vermieden, daß die Filme brüchig werden. Das fertig gerollte Stäbchen wird dann noch einmal einem Trocknungsprozeß unterworfen, um den gewünschten Gehalt an Restlösungsmittel einzustellen.

Implantate des Typs C und D und E werden vorteilhaft durch Extrusion oder Spritzgießen von Granulaten aus Wirkstoff und Polymer oder Copolymer, gegebenenfalls mit Zusätzen, wie z.B. Polymilchsäure, einen Weichmacher wie z.B. Triacetin oder einem Porenbildner, wie z.B. Laktose, hergestellt.

Die Wirkstofffreisetzung bei den ummantelten Typen C und E erfolgt aufgrund ihrer Konstruktion auf verschiedenen Wegen. Der im Kern der ummantelten Stäbchen des Typs C suspendierte Wirkstoff diffundiert durch Poren im Mantel, die durch Herauslösen - z.B. von Laktose - entstehen. Maßgebende Freigabefaktoren sind daher der Beladungsgrad des Mantels und die Partikelgröße der Laktose.

Im Gegensatz zu den ummantelten Formen des Typs C, die einen massiven wirkstoffhaltigen Kern enthalten und von einem "porösen" Mantel umhüllt sind, bestehen die Implantate des Typs E aus einem wirkstoffhaltigen Hohlzylinder (Schlauch), dessen Außenfläche von einem wirkstoffundurchlässigen Mantel umhüllt ist.

Bei dem Typ E kann der im schlauchförmigen Körper suspendierte Wirkstoff - wenn der Mantel porenfrei und undurchlässig ist - nur in den Hohlraum des Körpers (Schlauchs) freigesetzt werden. Bei diesem System werden die mit der Zeit länger werdenden Kanäle, d.h. Diffusionsstrecken, durch die Wirkstoffmenge in einem Segment kompensiert, die umso größer ist, je größer die Entfernung zur Zylinderachse ist.

## Figur A

Figur A zeigt einen Querschnitt durch die erfindungsgemäße Ausführungsform vom Typ E

Maßgebende Freigabefaktoren sind in diesem Falle neben dem Polymerabbau und dem Beladungsgrad die Abmessungen, wie z.B. die Länge und der innere Durchmesser des schlauchförmigen Implantates.

Es ist selbstverständlich, daß bei den Implantaten des Typs E der wirkstoffundurchlässige Mantel ebenfalls aus einem biologisch abbaubaren Polymer, bevorzugt einem Poly-D,L-Laktid, besteht. Ein wesentlicher Vorteil der so ausgebildeten Implantate ist, daß die Wirkstofffreisetzung nahezu linear erfolgt.

Implantate dieses Typs können auch auf Basis der zuvor beschriebenen Filme hergestellt werden, wobei der äußere Film aus einer wirkstoffreien, wirkstoffundurchlässigen Schicht besteht.

Die bisherigen Befunde zeigen, daß die nach der "Lösungsmittelmethode" hergestellten Implantate (Typ A und B) ein anderes Abbauverhalten zeigen als die Extrudate, d.h. die Extrudate werden bei gleicher Polymerzusammensetzung langsamer abgebaut (vgl. Abb. I). Der Unterschied beruht darauf, daß aufgrund der relativ hohen Temperaturen bei der Extrusion ein definierter, höherer Gehalt an Restlösungsmittel nicht eingestellt werden kann.

Erfindungsgemäße Wirkstoffabgabesysteme (Implantate), die durch Extrusions- oder Spritzgußverfahren hergestellt werden, können zweckmäßigerweise auf Basis eines Poly-D,L-Lactids mit einer inhärenten Viskosität zwischen 0.15 und 1.0 hergestellt werden. Polymere geringer Viskosität ($\eta$ = 0.15) können bereits unterhalb von 100 °C verarbeitet werden, was sich vorteilhaft auf die thermische Belastung der beigemischten Arzneistoffe auswirkt.

Implantate, die aus einem Poly-D,L-Lactid geringerer Viskosität hergestellt werden, weisen sowohl eine schnellere Freisetzung des Wirkstoffes wie auch einen rascheren Abbau des Implantats auf als dies bei höheren Viskositäten (> 0.3) der Fall ist, so daß ein Implantat gewünschtenfalls auch bereits nach 10 Wochen abgebaut sein kann.

Poly-D,L-Lactide geringerer Viskosität lassen sich durch partielle Hydrolyse aus Poly-D,L-Lactiden höherer Viskosität herstellen.

EP 0 311 065 B1

Die Wirkstoffreisetzung der erfindungsgemäßen Implantate kann durch einen zusätzlichen Überzug aus niedermolekularem Poly-D,L-Lactid, der keinen Wirkstoff enthält, aber wirkstoffdurchlässig ist, verzögert werden. Hierdurch wird verhindert, daß direkt nach der erfolgten Implantation in der Anfangsphase eine zu schnelle Freisetzung des Wirkstoffes erfolgt.

Geeignete Wirkstoffe sind solche, die in dem Polymer in suspendierter Form vorliegen. Besonders geeignet sind z.B. die wasserlöslichen Salzformen von Basen, wie z.B. Hydrochloride oder Hydrobromide. Ganz besonders geeignet ist Clenbuterolhydrochlorid.

Weiterhin können im Anwendungsbereich der Veterinärmedizin die nachfolgend genannten Stoffgruppen und Verbindungen in den erfindungsgemäßen Implantaten eingesetzt werden.

- Glucocorticoide zur Geburtsinduktion, z. B. Dexamethason, Betamethason, Flumethason, deren Ester und Derivate.
- Gestagene zur Brunstsynchronisation, Brunst- und Läufigkeitsunterdrückung,
- $\beta_2$-Adrenergika zur Therapie und Prophylaxe von Respirationserkrankungen, zur Verhinderung von Abort und Geburt, zur Wachstumsförderung und Stoffwechselbeeinflussung, wie z. B. Clenbuterol, 4-(2-tert,-Butylamino-1-hydroxyethyl)-2-cyano-6-fluor-phenylcarbaminsäureethylester-hydrochlorid, $\alpha$-[[-[3-(1-Benzimidazolyl)-1,1-dimethylpropyl]-amino]-methyl]-2-fluor-4-hydroxy-benzylalkoholmethansulfanat monohydrat, 1-(4-Amino-3-cyanophenyl)-2-iso-propylaminoethanol,
- $\beta$-Blocker zur MMA-Prophylaxe, zur Reduzierung von Transport-Stress, $\alpha_2$-Adrenergika gegen enteritische Erkrankungen und zur Behandlung hypoglykämischer Zustände, sowie zur Sedation (z. B. Clonidin, 2-[2-Brom-6-fluorphenylimino]-imidazolidin),
- Benzodiazepine und Derivate, wie z.B. Brotizolam zur Sedation,
- Antiphlogistika zur antiinflammatorischen Therapie, z.B. Meloxicam,
- Somatotropin und andere Peptidhormone zur Leistungssteigerung,
- Endorphine zur Anregung der Pansenmotorik,
- Steroidhormone (natürliche und synthetische) zur Wachstumsförderung, z.B. Östradiol, Progesteron und deren Ester und synthetische Derivate wie z.B. Trenbolon,
- Antiparasitika zur Bekämpfung von Endo- und Ektoparasiten, wie z.B. Avermectin,
- Herz- und Kreislaufaktive Substanzen, z.B. Etilefrin oder Pimobendan.

Die erfindungsgemäßen Implantate können im humanmedizinischen Bereich vorteilhaft zur Applikation von Hormonen insbesondere zur Empfängnisverhütung oder Cytostatika eingesetzt werden.

Es können Wirkstoffe verwandt werden, die sowohl eine systemische wie auch eine lokale Wirkung haben.

Ein bevorzugtes Anwendungsgebiet der erfindungsgemäßen Implantate ist die lokale Krebstherapie.

In den nachfolgenden Beispielen wird die Erfindung anhand von Beispielen näher erläutert.

In den Beispielen werden die nachfolgenden Polymere eingesetzt

An Polymermaterialien finden Verwendung:

$$\text{D,L-Polylactid I } [\eta] = 1,0 \ (\underline{\frac{100 \ ml}{g}} = MG^{*} = 123.000$$

$$\text{D,L-Polylactid II } [\eta] = 2,2 \quad " \qquad = MG = 300.000$$

$$\text{D,L-Polylactid III} \qquad\qquad MG = 11.500$$

$$\text{D,L-Polymilchsäure (MG = 2000)}$$

$$[\eta] = \text{Grenzviskosität (intrinsic viscosity)}$$

$^{*}$ bestimmt durch Gasphasenchromatographie
    (Standard: Polystyrol)

5

## Beispiel 1

(Faktoren des Polymerabbaues: Verarbeitungsmethode, Taktizität, Molmasse)

25 g D,L-Polylactid I werden in 75 g Essigester gelöst und mit einem Rakel auf einem glatten Untergrund zu einem Film ausgezogen. Nach Trocknung über mind.

24 Stunden wird dies zwei bzw. dreimal wiederholt, bis ein Mehrschichtenfilm von 250 $\mu$ Dicke entstanden ist. Anschließend wird der Film zuerst bei 23°C, dann bei 40°C im Vakuum bis zu einem vorgegebenen Lösungsmittelrestgehalt getrocknet, in 3 x 2,5 cm Stücke geschnitten und zu Rollen (Länge 3 cm, ∅ 2,8 mm) geformt.

Mittels Lösungsmethode hergestellte Implantate zeigen hinsichtlich Molmassenabnahme in einer Pufferlösung ein anderes Verhalten, als z.B. durch Extrusion gewonnene Implantate, d.h., sie werden vorteilhafterweise rascher abgebaut (Abb. 1). Die Taktizität des Polymeren spielt bei der Abbaugeschwindigkeit eine größere Rolle als die molare Masse bzw. Grenzviskosität $[\eta]$ (Abb. 2). Daß die in vitro Abbaugeschwindigkeit gut mit in vivo Werten übereinstimmt, zeigt Abb. 3.

Eine signifikante Massenabnahme setzt sowohl in vivo als auch in vitro nach ca. 70 Tagen ein, d.h. nachdem die Grenzviskosität auf einen Wert von $[\eta]$ 0,3 (100 ml/g) abgesunken ist (s. Abb. 4).

Die Applikation der Implantate bei Schaf, Ratte und Maus ließ im Beobachtungszeitraum (bis 140 Tage) keine besonderen Reaktionen erkennen, d.h. eine gute lokale Verträglichkeit der Implantate war gegeben (Tab. 1).

Statt durch die Lösungsmethode können entsprechend aufgebaute Formlinge auch durch Extrusion (Kern mit Mantel) von Granulaten aus Polymer, Wirk- und Zusatzstoffen hergestellt werden.

## Beispiel 2

(Faktoren des Polymerabbaues: Essigesterrestgehalt, Polymilchsäurezusatz)

Mehrschichtenfilmrollen werden, wie in Beispiel 1 beschrieben, hergestellt, wobei im Falle der Charge I 50 % des D,L-Polyactids durch D,L-Polymilchsäure (Molmasse 2000) ersetzt werden.

Abb. 5 zeigt, daß die Molmassenabnahme in wäßrigem Medium durch 4 bzw. 7 % Essigesterrestgehalt beschleunigt wird, nicht jedoch durch 1 %. Sehr ausgeprägt in dieser Beziehung wirkt der 50 %ige Zusatz von D,L-Polymilchsäure.

Die Massenabnahme verhält sich zur Molmassenabnahme wie in Beispiel 1 beschrieben (Abb. 6).

## Beispiel 3

(Faktor der Substanzfreigabe: Trägeraufbau)

25 g D,L-Polyactid II ($[\eta]$ = 2,2 (100 ml/g)) werden in 75 g Essigester gelöst, 5,0 g Methotrexat (MTX) Teilchengröße (30 $\mu$m = x = 60 $\mu$m) darin suspendiert und Dreischichtenfilme mit einer Schichtdicke von 0,80 mm analog Beispiel 1 hergestellt, wobei die obere und untere Polymerschicht wirkstoffrei bleibt. Nachdem der Lösungsmittelrestgehalt von 7 % erreicht ist, wird der Mehrschichtenfilm abweichend zum Beispiel 1 in Stäbchen von 1 x 1 x 10 mm geschnitten.

Aus solchen Implantaten wird MTX im Zeitraum von 10 bis ca. 60 Tagen sowohl in vivo als auch in vitro mit einer konstanten Geschwindigkeit von 63 $\mu$g/Tag freigesetzt, ohne daß die Polymermasse signifikant abnimmt (Abb. 7).

## Beispiel 4

(Faktor der Substanzfreigabe: Laktosezusatz)

8,8 g D,L-Polyactid II ($[\eta]$ = 2,2 (100 ml/g)) werden in 45 g Essigester gelöst und 2,7 g Clenbuterol. HCl (20 $\mu$ = x = 53 $\mu$m) suspendiert, und ein Dreischichtenfilm entsprechend Beispiel 1 hergestellt. Im Falle der Charge L wird in der Polymerlösung für die mittlere Schicht zusätzlich 25 Gew.% Laktose (1 - 5 $\mu$m) suspendiert.

Abb. 8 zeigt, daß sich durch den Laktosezusatz die Clenbuterolfreigabe in wäßrigem Medium beschleunigen und damit steuern läßt.

Beispiel 5

(Faktor der Substanzfreigabe: Polymilchsäurezusatz)

Die Dreischichtenfilmrolle L aus Beispiel 4 wird mit einer analog hergestellten Zubereitung verglichen, bei der 25 % des D,L-Polylactids II durch D,L-Polymilchsäure (Molasse 2000) ersetzt ist.

Während die Clenbuterolfreigabe in wäßrigem Medium durch den Polymilchsäurezusatz stark beschleunigt wird, blieb der Essigesterrestgehalt im Bereich von 1 - 4 % ohne Effekt auf das Freigabeverhalten.

Polymilchsäure kann demnach ebenso wie Laktose als ein die Freigabe steuernder Zusatz eingesetzt werden.

Beispiel 6

(Faktor der Substanzfreigabe: Trägeraufbau)

(Ausführungsform E)

D,L-Polylactid III ohne Wirkstoff und ein Schmelzgranulat aus 3 Gew. Teilen D,L-Polylactid III und 1 Gew. Teil Clenbuterol (Hydrochlorid, 20 - 53 $\mu$m) werden bei 90° C (Massentemperatur) zu einem doppelwandigen Schlauch verarbeitet (das ist sowohl mittels eines geeigneten Extruders als auch durch Spritzgußtechnik möglich).

Ein Implantat der Ausführungsform E - hergestellt nach Beispiel 6 - folgender Abmessungen wurde für in vitro Versuche bzgl. Polymerabbau und Wirkstofffreigabe verwendet: Länge: 10 mm; Hohlraum: Durchmesser - 2 mm; Gesamtdurchmesser: 5 mm; äußerer Mantel: wirkstoffrei, undurchlässig; Wandstärke: 0,5 mm; innerer Schlauch: wirkstoffhaltig, Wandstärke: 1,0 mm.

Abb. 10 beschreibt den nahezu linearen Polymermassenabbau in vitro mit einer Halbwertszeit von ca. 70 Tagen, Abb. 11 die nahezu lineare Clenbuterolfreigabe.

Tabelle I    <u>Pathologie, Histologie der Implantate</u>

| | | Befunde | |
|---|---|---|---|
| | | <u>bis ca. 60 Tage:</u> leichte Kapsel-bildung, leichte Ent-zündungen, übliche Makro-phagenbildung | <u>ab ca.100 Tage:</u> leichte Narben-bildung, keine Ent-zündungen oder keine sonst. Befunde |
| Spezies | Appli-kation | vereinzelt Zelldetritus | |
| Schaf | s.c., hinter dem Ohr | Reaktion normal | ja |
| Maus | s.c. Hals s.c. Rücken | Reaktion normal Reaktion normal | ja ja |
| Ratte | intra-cerebal | Reaktion normal | ja |
| | intra-tumoral, Rücken | Reaktion normal | ja |

Abb. 1: Molmassenabnahme von Polylactid-Implantaten

$[\eta]$ = Grenzviskosität

Versuchsbedingungen in vitro: isoton. Phosphatpuffer pH 7,4; 37 °C

A: Gerolltes Scäbchen aus D,L-Polylactid I (Lösungsmethode)

B: Extrudatzylinder aus D,L-Polylactid I

C: D,L-Polylactid I-Pulver

Abb. 2 Molmassenabnahme von Polylactid-Implantaten

Versuchsbedingungen in vitro: isotonischer Phosphatpuffer pH 7.4; 37°C
Zubereitung: Gerollte Stäbchen aus Polylaktid (Lösungsmethode)
A:     D,L-Polylactid I; 7 % Essigester, Tg = 26°C
D:     D,L-Polylactid II; 7 % Essigester. Tg = 30°C
E:     L-Polylactid; 7 % Essigester, Fp = 172°C (Vergleichsbeispiel)

Abb. 3: Molmassenabnahme von D,L-Polylactid-Implantaten

Zubereitung: Mehrschichtenfilmrollen, Charge D
    A. Applikation: in vivo, Schaf, s.c.

B. in vitro, Versuchsbedingung: isoton. Phosphatpuffer; pH 7,4; 37 ° C

Abb. 4: Massenabnahme von Polylactid-Implantaten

Zubereitung: Filmrollen aus D,L-Polylactid ([$\eta$] = 2,19 (100 ml/g);
Charge D
    A. in vitro Versuchsbedingungen: isotoner Phosphatpuffer; pH 7,4; 37 ° C

B. Applikation: in vivo, Schaf, s.c.

Abb. 5 Molmassenabnahme von Polylactid-Implantaten

Versuchsbedingungen in vitro: isotonischer Phosphatpuffer; pH 7,4; 37°C
Zubereitung: Filmrollen Lösungsmethode)

A:    D,L-Polylactid I; 7 % Essigester; Tg = 26°C
F:    D,L-Polylactid I; 1 % Essigester; Tg = 48°C
G:    D,L-Polylactid I; 4 % Essigester; Tg = 35°C
H:    D,L-Polylactid II; 1 % Essigester; Tg = 52°C
I:    D,L-Polylactid II +
         50 % D,L-Polymilchsäure;
         1 % Essigester; Tg = 30°C

Abb. 6: Massenabnahme von D,L-Polylactid-Implantaten

in vitro Versuchsbedingungen: isot. Phosphatpuffer; pH 7,4 %; 37 ° C
Zubereitung: Filmrollen (Lösungsmethode)

A:      D,L-Polylactid I; 7 % Essigester; Tg = 26 ° C
F:      D,L-Polylactid I; 1 % Essigester; Tg = 48 ° C
G:      D,L-Polylactid I; 4 % Essigester; Tg = 35 ° C
H:      D,L-Polylactid II; 1 % Essigester; Tg = 52 ° C
I:      D,L-Polylactid II + 50 % D,L-Polymilchsäure
            1 % Essigester; Tg = 30 ° C

Abb. 7: Methotrexatfreigabe (MTX) aus Polylactid-Implantaten

Zubereitung: Mehrschichtenstäbchen aus D,L-Polylactid ($[\eta]$ = 2,2 (100 ml /g)),
A. Applikation: Ratte, intracerebral

B. in vitro Bedingungen: isoton. Phosphatpuffer; pH 7,4; 37 ° C

Clenbuterol-Gehalt (%)

Abb. 8: Clenbuterol-Freigabe aus D,L-Polylactid-Implantaten

in vitro Versuchsbedingungen: isoton. Phosphatpuffer; pH 7,4; 37 ° C
Zubereitung: 3-Schichten-Filmrollen ($[\eta]$ = 2,2 (100 ml/g)) mit 23,5 Gew. % Clenbuterol HCl und 4 % Essigester

|   | Laktose (Gew. %) | | |
|---|---|---|---|
|   | 1. Schicht | 2.Schicht | 3. Schicht |
| K | 0 | 0 | 0 |
| L | 0 | 25 % | 0 |

Abb. 9 Clenbuterol-Freigabe aus D,L-Polylactid-Implantaten

in vitro Versuchsbedingungen isoton. Phosphatpuffer; pH 7,4; 37°C.

Zubereitung: 3-Schicht-Filmrollen mit 10 Gew.% Laktose und 23,5 Gew. % Clenbuterol HCl D,L-Polylactid II ($[\eta]$ = 2,2 (100 ml/g))

Zusätze: L: 4 % Essigester
M: 1 % Essigester
N: 1 % Essigester + 25 % D,L-Polymilchsäure

Polymermasse (%)

18

Abb. 10 Massenabnahme von Polylactid-Implantaten

Zubereitung:　　Doppelwandiges schlauchförmiges Implantat aus D,L-Polylactid III (vgl. Beispiel 6)
in vitro Versuchsbedingungen: isotoner Phosphatpuffer pH 7, 4, 37 ° C

Abb. 11 Clenbuterol-Freigabe aus D,L-Polylactid-Implantaten

Zubereitung:　　Doppelwandiges schlauchförmiges Implantat aus D,L-Polylactid III (vgl. Beispiel 6)
in vitro
Versuchsbedingungen:　　isotoner Phosohatpuffer pH 7, 4, 37 ° C

## Patentansprüche

1. Implantierbares, biologisch abbaubares Wirkstoffreigabesystem, bestehend aus einem Trägermaterial auf der Basis von Poly-D,L-Lactid und einem darin eingearbeiteten Wirkstoff sowie gegebenenfalls pharmazeutischen Hilfsstoffen, dadurch gekennzeichnet, daß das Trägermaterial definierte Anteile an Zusätzen in Form von Porenbildnern, bis 10 Gew.-% Essigsäureester und/oder Polymilchsäure enthält.

2. Wirkstoffreigabesystem nach Anspruch 1, dadurch gekennzeichnet, daß das Trägermaterial aus einem Copolymerisat aus D,L-Lactid und Glycolid besteht, wobei der Glycolidanteil 50 Gew.% nicht überschreitet.

3. Wirkstoffreigabesystem nach Anspruch 1 oder 2, dadurch gekennzeichnet, daß das Trägermaterial bis zu 10 Gew.-% Essigsäureethylester enthält.

**4.** Wirkstoffreigabesystem nach einem der Ansprüche 1 bis 3, dadurch gekennzeichnet, daß das Trägermaterial niedermolekulare Polymilchsäure und/oder Lactose enthält.

**5.** Wirkstoffreigabesystem nach einem der Ansprüche 1 bis 4, dadurch gekennzeichnet, daß es die Form eines Stäbchens, gegebenenfalls mit Schichtaufbau, aufweist.

**6.** Wirkstoffreigabesystem nach Anspruch 5, dadurch gekennzeichnet, daß es aus mindestens einem, zu einem Stäbchen aufgerollten, gegebenenfalls mehrschichtigen Film besteht.

**7.** Wirkstoffreigabesystem nach Anspruch 5, dadurch gekennzeichnet, daß es aus einem mehrschichtigen, aufgerollten Film unterschiedlicher Zusammensetzung besteht.

**8.** Wirkstoffreigabesystem nach Anspruch 5, dadurch gekennzeichnet, daß das Stäbchen, gegebenenfalls mit Mantelschichten, durch Extrusion hergestellt ist.

**9.** Implantierbares, biologisch abbaubares Wirkstoffreigabesystem gemäß einem der Ansprüche 1 bis 4, dadurch gekennzeichnet, daß es aus einem wirkstoffhaltigen Hohlzylinder (Schlauch) und aus einer äußeren wirkstoffundurchlässigen Umhüllung besteht, so daß der Wirkstoff über den Hohlraum des Zylinders freigesetzt wird.

**10.** Verfahren zur Herstellung eines Wirkstoffreigabesystems nach einem der Ansprüche 1 bis 7, dadurch gekennzeichnet, daß man die Wirkstoff und Zusätze enthaltende Lösung des Polymers zu einem Film ausgießt, dann trocknet und anschließend den Film in entsprechende Stäbchen schneidet

**11.** Verfahren zur Herstellung eines Wirkstoffreigabesystems nach einem der Ansprüche 1 bis 7, dadurch gekennzeichnet, daß man die Wirkstoff und Zusätze enthaltende Lösung des Polymers zu einem Film ausgießt, auf einen Restlösungsmittelgehalt von 10 % trocknet, gegebenenfalls weitere Filmschichten aufgießt und trocknet, den Film zu Stäbchen rollt und anschließend die Stäbchen so trocknet, daß der gewünschte Gehalt an Restlösungsmittel erhalten wird.

**12.** Verfahren zur Herstellung eines Wirkstoffreigabesystems gemäß Anspruch 7 nach Anspruch 10 oder 11, dadurch gekennzeichnet, daß mindestens zwei Filme unterschiedlicher Zusammensetzung zu einem Stäbchen gerollt werden.

**13.** Verfahren zur Herstellung eines Wirkstoffreigabesystems nach einem der Ansprüche 1 bis 5, 8 oder 9, dadurch gekennzeichnet, daß ein Granulat bestehend aus dem Polymer, dem Wirkstoff und den Zusätzen in Form von gegebenenfalls ummantelten Schläuchen oder Stäbchen extrudiert wird, die dann auf die gewünschte Länge geschnitten werden.

**14.** Verwendung eines Implantats nach einem der Ansprüche 1 bis 9 in der Tiermedizin.

**15.** Implantat nach einem der Ansprüche 1 bis 9 dadurch gekennzeichnet, daß es als Wirkstoff ein Hormon oder ein Cytostatikum enthält.

**Claims**

**1.** Implantable biodegradable system for releasing active substance, consisting of a carrier material based on poly-D,L-lactide and an active substance incorporated therein and optionally pharmaceutical excipients, characterised in that the carrier material contains defined amounts of additives in the form of pore-forming agents, up to about 10% by weight of an acetic acid ester and/or polylactic acid.

**2.** System for releasing active substance according to claim 1, characterised in that the carrier material consists of a copolymer of D,L-lactide and glycolide, in which the glycolide content does not exceed 50% by weight.

**3.** System for releasing active substance according to claim 1 or 2, characterised in that the carrier material contains up to 10% by weight of ethyl acetate.

**4.** System for releasing active substance according to one of claims 1 to 3, characterised in that the carrier material contains low molecular polylactic acid and/or lactose.

**5.** System for releasing active substance according to one of claims 1 to 4, characterised in that it takes the form of a rod, optionally of layered structure.

**6.** System for releasing active substance according to claim 5, characterised in that it consists of at least one, optionally multi-layered, film rolled up to form a rod.

**7.** System for releasing active substance according to claim 5, characterised in that it consists of a multi-layer rolled film of different composition.

**8.** System for releasing active substance according to claim 5, characterised in that the rod, optionally with outer layers, is produced by extrusion.

**9.** Implantable biodegradable system for releasing active substance according to one of claims 1 to 4, characterised in that it consists of a hollow cylinder (tube) containing the active substance and an outer casing impermeable to the active substance, so that the active substance is released through the cavity in the cylinder.

**10.** Process for preparing a system for releasing active substance according to one of claims 1 to 7, characterised in that the solution of the polymer containing the active substance and additives is poured out to form a film, then dried and then the film is cut into suitable strips.

**11.** Process for preparing a system for releasing active substance according to one of claims 1 to 7, characterised in that the solution of the polymer containing the active substance and additives is poured out to form a film, dried to a residual solvent content of 10%, if desired further layers of film are poured out and dried, the film is rolled up to form rods and the rods are then dried so as to achieve the desired residual solvent content.

**12.** Process according to claim 10 or 11 for preparing a system for releasing active substance according to claim 7, characterised in that at least two films of different composition are rolled up to form a rod.

**13.** Process for preparing a system for releasing active substance according to one of claims 1 to 5, 8 or 9, characterised in that granules consisting of the polymer, the active substance and the additives are extruded in the form of optionally encased tubes or rods which are then cut to the desired length.

**14.** Use of an implant according to one of claims 1 to 9 in veterinary medicine.

**15.** Implant according to one of claims 1 to 9, characterised in that it contains as active substance a hormone or a cytostatic agent.

**Revendications**

**1.** Système implantable et biodégradable de libération de principe actif, consistant en un matériau support à base de poly-D,L-lactide et en un principe actif incorporé dans celui-ci, et éventuellement en adjuvants pharmaceutiques, caractérisé en ce que le matériau support contient des proportions définies d'additifs sous forme d'agents porogènes, jusqu'à 10% en poids d'acétate et/ou de poly(acide lactique).

**2.** Système de libération de principe actif selon la revendication 1, caractérisé en ce que le matériau support consiste en un copolymère de D,L-lactide et de glycolide dans lequel la proportion de glycolide ne dépasse pas 50% en poids.

**3.** Système de libération de principe actif selon la revendication 1 ou 2, caractérisé en ce que le matériau support contient jusqu'à 10% en poids d'acétate d'éthyle.

**4.** Système de libération de principe actif selon l'une des revendications 1 à 3, caractérisé en ce que le matériau support contient du poly(acide lactique) de faible masse moléculaire et/ou du lactose.

**5.** Système de libération de principe actif selon l'une des revendications 1 à 4, caractérisé en ce qu'il est sous forme d'un bâtonnet présentant éventuellement une structure en couches.

**6.** Système de libération de principe actif selon la revendication 5, caractérisé en ce qu'il consiste en au moins un film, éventuellement à couches multiples, enroulé en bâtonnet.

**7.** Système de libération de principe actif selon la revendication 5, caractérisé en ce qu'il consiste en un film enroulé à couches multiples de composition différente.

**8.** Système de libération de principe actif selon la revendication 5, caractérisé en ce que le bâtonnet, qui comporte éventuellement des couches d'enveloppe, est fabriqué par extrusion.

**9.** Système implantable et biodégradable de libération de principe actif selon l'une des revendications 1 à 4, caractérisé en ce qu'il consiste en un cylindre creux (tuyau) contenant le principe actif et en une enveloppe externe imperméable au principe actif de sorte que le principe actif est libéré par l'intermédiaire de la cavité du cylindre.

**10.** Procédé de fabrication d'un système de libération de principe actif selon l'une des revendications 1 à 7, caractérisé en ce que l'on coule en un film la solution de polymère contenant le principe actif et les additifs, puis on la sèche et on coupe le film en les bâtonnets correspondants.

**11.** Procédé de fabrication d'un système de libération de principe actif selon l'une des revendications 1 à 7, caractérisé en ce que l'on coule en un film la solution de polymère contenant le principe actif et les additifs, on la sèche à une teneur en solvant résiduel de 10%, éventuellement on coule dessus d'autres couches de film que l'on sèche, on enroule le film en bâtonnets puis on sèche les bâtonnets de manière à obtenir la teneur voulue en solvant résiduel.

**12.** Procédé de fabrication selon la revendication 10 ou 11 d'un système de libération de principe actif selon la revendication 7, caractérisé en ce qu'au moins deux films de composition différente sont enroulés en un bâtonnet.

**13.** Procédé de fabrication d'un système de libération de principe actif selon l'une des revendications 1 à 5, 8 ou 9, caractérisé en ce qu'un granulat consistant en le polymère, le principe actif et les additifs est extrudé sous forme de tuyaux ou bâtonnets éventuellement enveloppés, qui sont ensuite coupés à la longueur voulue.

**14.** Utilisation d'un implant selon l'une des revendications 1 à 9 en médecine vétérinaire.

**15.** Implant selon l'une des revendications 1 à 9 caractérisé en ce qu'il contient comme principe actif une hormone ou un cytostatique.

22